# EUROPÄISCHE PATENTANMELDUNG

(11) **EP 1 920 736 A1**
(43) Veröffentlichungstag der Anmeldung: **14.05.2008**
(21) Anmeldenummer: 07117922.0
(22) Anmeldetag: 04.10.2007
(51) Int. Cl.: A61F 2/36

(54) **Gelenkkopf-Kappenimplantat für ein künstliches Hüftgelenk**

(30) Priorität: 07.11.2006 DE 202006017005 U
(71) Anmelder: ESKA-medical Gesellschaft für Entwicklung und Vertrieb von medizinischen Implantat-Artikeln mbH, 23556 Lübeck (DE)
(72) Erfinder: Grundei, Hans, 23558, Lübeck (DE)
(74) Vertreter: Fuchs

(57) **Zusammenfassung**

Die Erfindung betrifft ein Gelenkkopf-Kappenimplantat für ein künstliches Hüftgelenk aus einer dünnwandigen metallischen Kappe (1) zum Aufsatz auf den natürlichen, lediglich entknorpelten oder angefrischten Hüftgelenkskopf mit einem proximal im Polbereich der Kappe (1) exakt mittig angeordneten Führungsstift (2).

## Beschreibung

Die Erfindung betrifft ein Gelenkkopf-Kappenimplantat für ein künstliches Hüftgelenk, und zwar als ein Oberflächenersatz für die natürliche Artikulationsfläche des Hüftgelenkkopfes.

In jüngster Zeit kommen verstärkt so genannte Kappenimplantate zur Anwendung, welche über den präparierten natürlichen Restgelenkkopf des Hüftgelenkes gesetzt werden und in dieser Lage dann fixiert werden können. Kappenimplantate bestehen aus einer der äußeren Form der natürlichen Gelenkkugel nachgebildeten Kappe, die auf einen (teil-) präparierten natürlichen Restgelenkkopf setzbar ist.

Voraussetzung für eine stabile Sekundärfixation ist stabiles Knochenmaterial des Restknochens. So wurde in der DE-C-1 02 18 801 vorgeschlagen, an die Gelenkkopfkappe einen Zapfen anzukoppeln, der in eine entsprechende Ausfräsung im Schenkelhals gesetzt wird. Dieser Zapfen weist eine Oberfläche auf, welche mit einer dreidimensionalen offenmaschigen Raumnetzstruktur versehen ist, in welche und durch welche hindurch Knochentrabekel des umliegenden Knochenmaterials wachsen und für die stabile Sekundärfixation sorgen.

Es gibt jedoch Indikationen, bei denen man noch davon absehen kann, den Schenkelhals auszufräsen, um so Platz für den Zapfen zu schaffen.

Angeführt wird hier die so genannte Perthes-Calvé-Legg-Krankheit, die ein- oder beidseitig im Bereich der Femurkopfepiphyse aseptische Knochennekrosen hervorruft. Vor allem bei Jungen vom vierten bis zwölften Lebensjahr tritt diese Krankheit auf (Pschyrembel Klinisches Wörterbuch, 259. Auflage, 2002, Seite 1285). Eine Ausheilung ohne Deformierung ist zwar möglich, jedoch bleibt eine eventuelle Walzen- oder Pilzform des Schenkelkopfes mit Abplattung der Gelenkpfanne, seltener Coxa plana oder Arthrosis deformans zurück.

Eine weitere Indikation ist beispielsweise eine Zyste im Hüftgelenkskopf, die zu Oberflächendefekten des Gelenkkopfes führt.

Generell kann eine Nekrose des Gelenkkopfes zu oberflächenhaften Defekten führen, die es aber immer noch nicht rechtfertigen, den Gelenkkopf vollständig zu resezieren und den Patienten mit einer Kurzstielendoprothese (EP 0 878 176) zu versorgen.

Grundsätzlich - und dies wurde in letzter Zeit verstärkt erkannt - ist es vorteilhaft, mit (Teil-)resektionen von Knochen solange wie möglich zu zögern, um bei einem eventuell später notwendigen Revisionseingriff auf mehrere Stufen der endoprothetischen Versorgung zurückgreifen zu können, von der Kurzstielendoprothese bis zur Langstielendoprothese. Der Einsatz der letztgenannten Endoprothese erfordert die vollständige Resektion des Schenkelhalses.

Der Zapften gemäß der schon erwähnten DE-C-102 18 801 hat eine tragende und fixierende Funktion. Problematisch ist dabei jedoch die achsgerechte Positionierung der Kappe auf dem präparierten Restgelenkkopf. Eine Fehlstellung kann später dann zu weit reichenden Konsequenzen führen.

Vor diesem Hintergrund ist es die Aufgabe der vorliegenden Erfindung ein Gelenkkopf-Kappenimplantat anzugeben, bei dem das daraus erstellte Gelenkkopf-Kappenimplantat achsgerecht auf dem natürlichen Gelenkkopf positioniert werden kann. Der natürliche Gelenkkopf soll dabei weitgehend intakt bleiben.

Gelöst wird diese Aufgabe durch das Kappenimplantat mit den Merkmalen des Anspruchs 1. Weitere vorteilhafte Ausführungsformen ergeben sich aus den Unteransprüchen.

Demnach besteht das Kappenimplantat für ein künstliches Hüftgelenk aus einer dünnwandigen metallischen Kappe zum Aufsatz auf den natürlichen, lediglich entknorpelten oder angefrischten Hüftgelenkskopf mit einem proximal im Polbereich der Kappe exakt mittig angeordneten Führungsstift.

Der knöcherne Hüftgelenkskopf wird nicht mit einem Formfräser bearbeitet in dem Sinne, dass die bislang üblichen konischen Strukturen unter Knochenzurichtung ausgebildet werden, wie dies bei der Implantation eines üblichen Kappenimplantates der Fall ist. Der Gelenkkopf wird lediglich von Knorpel und Bindegewebe befreit, d.h. entknorpelt. Lediglich in einem Grenzfall, bei der die äußerste Schicht des Hüftgelenkkopfes leichte Schäden aufweist, wird der Hüftgelenkkopf soweit angefrischt, dass die Spongiosa gerade sichtbar wird. Selbst in diesem Fall behält der Gelenkkopf jedoch seine natürliche Grundform. Für den Fall der Entknorpelung wird die Kappe sodann mit Zement über den Gelenkkopf gezogen und dort durch die Abbindung des Zementes fixiert. Im Falle eines angefrischten Hüftgelenkkopfes ist auch eine zementlose Fixiation möglich, wenn nämlich im Kappeninneren Strukturen ausgebildet sind, die ein Ein- und Durchwachsen von Knochentrabekeln ermöglichen.

Die Materialstärke der Kappe liegt vorzugsweise zwischen etwa 1 mm und 1,5mm. Diese Materialstärke wird als Ausgleichsstärke angesehen und zwar für das entfernte Knorpel- und Bindegewebe. Bei der Implantation wird entlang der Achse des Schenkelhalses eine Bohrung in den Knochen eingebracht mit einem Durchmesser, der dem Durchmesser des Führungsstiftes entspricht oder geringfügig größer ist.

Sodann wird das Kappenimplantat zuerst mit dem Führungsstift in die erwähnte Bohrung gesetzt und schließlich über den Hüftgelenkkopf geschoben. Hierbei übt der Führungsstift seine Funktion aus. Wenn das Implantat auf dem Hüftgelenkskopf positioniert ist, hat der Führungsstift keine weitere Funktion mehr, insbesondere keine ablastende oder fixierende Funktion.

Gemäß einer besonders bevorzugten Ausführungsform ist der Führungsstift einstückig mit der Kappe ausgebildet.

Es gibt Gelenkkopf-Kappenimplantate gemäß der vorliegenden Erfindung mit unterschiedlichen Längen des Führungsstiftes. Die Wahl des richtigen Implantates obliegt dem Operateur, der patienten- und indikationsbedingt ein Implantat mit angemessener Länge des Führungsstiftes auswählt.

Bei der Indikation einer Osteoporose des Schenkelhalses genügt eine reine Führung des Führungsstiftes gemäß der vorerwähnten Lösung nicht mehr. Vielmehr bedarf der ermüdende Schenkelhals einer Unterstützung. Gemäß einer alternativen Lösung ist daher ein Gelenkkopf-Kappenimplantat für ein künstliches Hüftgelenk aus einer dünnwandigen metallischen Kappe zum Aufsatz auf den natürlichen, lediglich entknorpelten oder angefrischten Hüftgelenkskopf mit einem proximal im Polbereich der Kappe exakt mittig angeordneten Führungs- und Ablastelement vorgesehen, welches über die Außenkonturen der Kappe weit hinausragt, so dass es nämlich nach der Implantation in den Schenkelhals hineinragt. Vor der Implantation wird wiederum entlang der Achse des Schenkelhalses eine (größere) Bohrung in den Knochen eingebracht. Sodann wird das Kappenimplantat zuerst mit dem Führungs- und Ablastelement in die erwähnte Bohrung gesetzt und schließlich über den Hüftgelenkkopf geschoben. Hierbei übt das Führungs- und Ablastelement seine Funktion aus. Wenn das Implantat auf dem Hüftgelenkskopf positioniert ist, hat das Element weiterhin eine ablastende und unterstützende Funktion inne. Das Führungs- und Ablastelement gemäß dieser zweiten Lösung ist wesentlich stärker ausgeführt als der Führungsstift gemäß dem ersten Lösungsvorschlag, der aber - wie ausgeführt - nach Implantation keinerlei Funktion mehr ausübt.

Auch das Kappenimplantat gemäß der zweiten Lösung kann so ausgebildet sein, dass das Führungs- und Ablasselement einstückig mit der Kappe ausgebildet ist.

Bevorzugt wird eine Weiterbildung, bei der im Inneren der Hüftgelenkskopfkappe wenigsten zwei Antirotationselemente in das Kappeninnere hineinragen. Dies erhöht die Sicherheit des Sitzes der Kappe auf dem Hüftgelenkskopf. Die Antirotationselemente greifen nach der Implantation in den Knochen des Hüftgelenkkopfes hinein und verhindern so ein Drehen der Kappe auf dem Gelenkkopf.

Gemäß einer konkreten Ausführungsform sind die Antirotationselemente schildförmig ausgebildet. Alternativ können sie zapfenförmig ausgebildet sein.

Die Erfindung wird anhand der Zeichnungsfiguren beispielhaft näher erläutert. Hierbei zeigt:
- Fig. 1: verschiedene Ansichten gemäß einer ersten Lösung des Kappenimplantates, nämlich im Schnitt (a), perspektivisch von links unten gesehen (b) und die Ansicht (c) des Kappeninneren, und
- Fig. 2: ähnliche Ansichten eines Kappenimplantates gemäß einer zweiten Lösung wie in Fig. 1, nämlich im Schnitt (a), perspektivisch von links unten gesehen (b) und die Ansicht (c) des Kappeninneren.

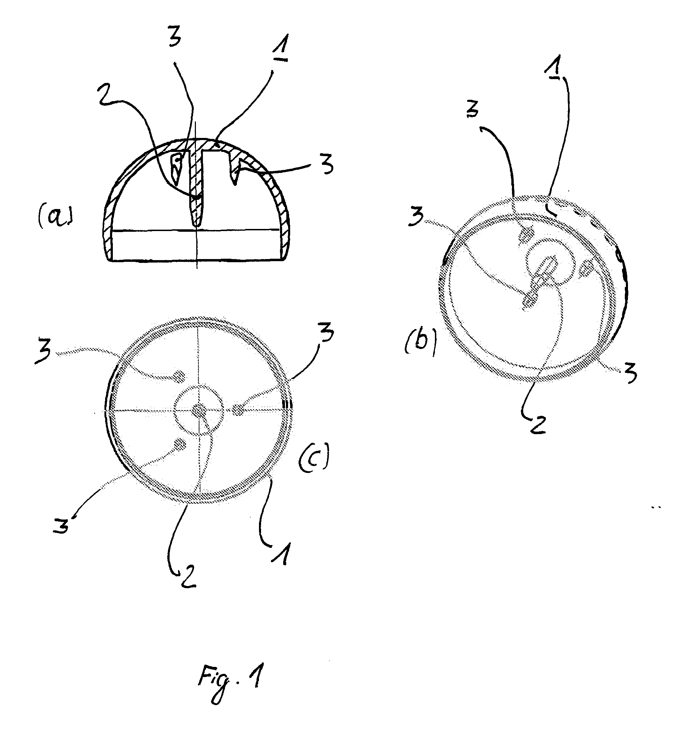

Fig. 1(a) zeigt eine Schnittansicht eines Kappenimplantates. Zu erkennen ist die dünnwandige Kappe 1 (aus Metall) zum Aufsatz auf den natürlichen, lediglich entknorpelten oder angefrischten Hüftgelenkskopf, der sich in Igluform darstellt.

Wie deutlich erkennbar, ist proximal im Polbereich der Kappe 1 ein exakt mittig angeordneter Führungsstift 2 vorgesehen. Dieser ist im vorliegenden Falle einstückig mit der Kappe 1 ausgebildet. Der Führungsstift 2 hat vorliegend keine tragende und fixierende Funktion. Er dient lediglich der Führung beim Aufsetzen der Kappe 1 auf den entknorpelten oder angefrischten Hüftgelenkskopf.

Im Inneren der Kappe 1 sind drei Antirotationselemente 3 angeordnet, die vorliegend zapfenförmig ausgebildet sind. Die Antirationselemente 3 greifen in den Knochen des Hüftgelenkkopfes hinein und verhindern ein Drehen der Kappe auf dem Hüftgelenkskopf.

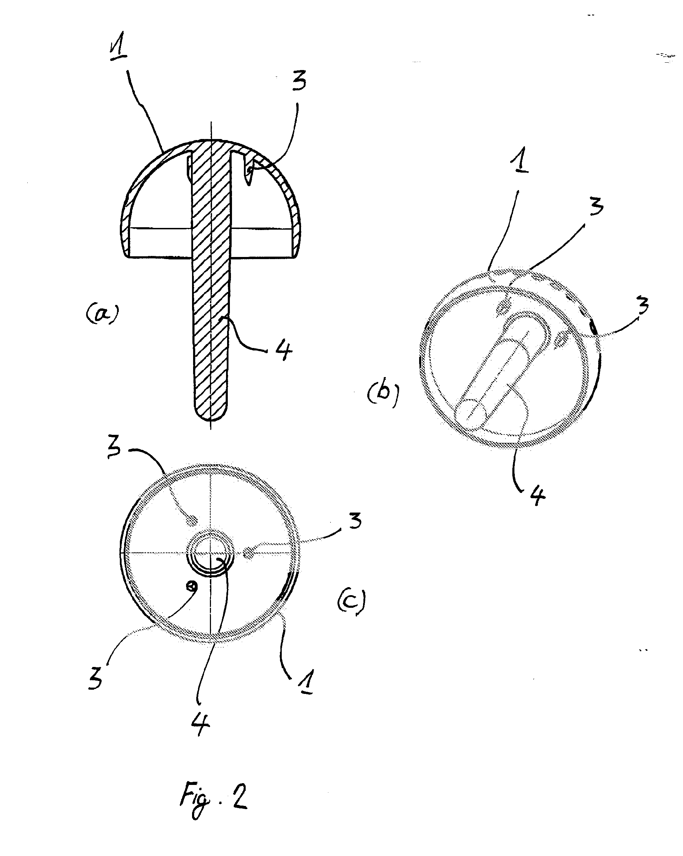

In Fig. 2 ist eine andere Form des Kappenimplantates dargestellt, und zwar mit einem Führungs- und Ablastelement 4. Auch in diesem Falle ist das proximal im Polbereich der Kappe 1 exakt mittig angeordnete Führungs- und Ablastelement 4 einstückig mit der Kappe 1 ausgebildet. Auch vorliegend sind wiederum drei Antirotationselemente 3 zur Sicherung des Sitzes der Kappe 1 auf dem Hüftgelenkskopf vorgesehen.

Im Unterschied zu der Ausführungsform gemäß Fig. 1 weist das Kappenimplantat vorliegend ein wesentlich größeres Führungs- und Ablastelement 4 im Kappeninneren auf. Dieses dient sowohl der Führung der Kappe 1 beim Aufsetzen auf den Hüftgelenkskopf, als auch dem Ablasten von Belastungskräften, um so das osteoporotische Gewebe im Schenkelhals zu entlasten.

## Patentansprüche

1. Gelenkkopf-Kappenimplantat für ein künstliches Hüftgelenk aus einer dünnwandigen metallischen Kappe (1) zum Aufsatz auf den natürlichen, lediglich entknorpelten oder angefrischten Hüftgelenkskopf mit einem proximal im Polbereich der Kappe (1) exakt mittig angeordneten Führungsstift (2).

2. Kappenimplantat nach Anspruch 1, bei dem der Führungsstift (2) einstückig mit der Kappe (1) ausgebildet ist.

3. Gelenkkopf-Kappenimplantat für ein künstliches Hüftgelenk aus einer dünnwandigen metallischen Kappe (1) zum Aufsatz auf den natürlichen, lediglich entknorpelten oder angefrischten Hüftgelenkskopf mit einem proximal im Polbereich der Kappe (1) exakt mittig angeordneten Führungs- und Ablastelement (4), welches über die Außenkontur der Kappe (1) weit hinausragt.

4. Kappenimplantat nach Anspruch 3, bei dem das Führungs- und Ablastelement (4) einstückig mit der Kappe (1) ausgebildet ist.

5. Kappenimplantat nach einem der Ansprüche 1 bis 4, bei dem im Inneren der Kappe (1) wenigstens zwei Antirotationselemente (3) in das Kappeninnere hineinragen.

6. Kappenimplantat nach Anspruch 5, bei dem die Antirotationselemente (3) schildförmig ausgebildet sind.

7. Kappenimplantat nach Anspruch 5, bei dem die Antirotationselemente (3) zapfenförmig ausgebildet sind.
